# EUROPÄISCHE PATENTSCHRIFT

(11) **EP 2 814 978 B1**
(45) Veröffentlichungstag und Bekanntmachung des Hinweises auf die Patenterteilung: **13.01.2016**
(21) Anmeldenummer: 13703454.2
(22) Anmeldetag: 12.02.2013
(51) Int. Cl.: C12Q 1/68, C12M 1/34, C12Q 1/04

(54) **ANORDNUNG UND VERFAHREN ZUM NACHWEIS VON MIKROORGANISMEN IN EINEM KULTURGEFÄSS**
ARRANGEMENT AND METHOD FOR DETECTING MICROORGANISMS IN A CULTURE VESSEL
SYSTÈME ET PROCÉDÉ DESTINÉS À DÉTECTER DES MICRO-ORGANISMES DANS UN RÉCIPIENT DE CULTURE

(30) Priorität: 13.02.2012 AT 1832012
(43) Veröffentlichungstag der Anmeldung: 24.12.2014
(73) Patentinhaber: Greiner Bio-One GmbH, 72636 Frickenhausen (DE)
(72) Erfinder: MITTERMAYR, Christian Rudolf, A-4111 Walding (AT); WINKLEHNER, Peter Johannes, A-4261 Rainbach (AT)
(74) Vertreter: Burger, Hannes
(86) Internationale Anmeldenummer: PCT/EP2013/052791
(87) Internationale Veröffentlichungsnummer: WO 2013/120853

(56) Entgegenhaltungen:
- WO-A1-2006/092629
- WO-A1-2009/049833
- WO-A2-03/014382
- WO-A2-2004/106547
- US-A1- 2003 039 974

## Beschreibung

Die Erfindung betrifft eine Anordnung, insbesondere Hybridisierungsanordnung, zum spezifischen Nachweis von Mikroorganismen in einem Kulturgefäß mit zumindest zwei Bereichen sowie deren Verwendung und ein Verfahren zum spezifischen Nachweis von Mikroorganismen mit einer Anordnung, insbesondere Hybridisierungsanordnung, in einem Kulturgefäß mit zumindest zwei Bereichen vorzugsweise durch Hybridisierung.

Der Nachweis von Mikroorganismen erfolgte und erfolgt auch heutzutage meist mittels Kultivierung. Hierbei werden Mikroorganismen auf ein künstliches Nährmedium überführt und auf diesem bei einer bestimmten, optimalen Wachstumstemperatur bebrütet und nach dem Wachstum wird die Bakterienkolonie identifiziert. Die Identifizierung kann mittels morphologischer, chemischer oder physiologischer Merkmale erfolgen. Das Ergebnis ist oft nicht immer eindeutig und liegt meist erst nach mehreren Tagen vor.

Ein Verfahren zum molekularbiologischen und somit eindeutigen Nachweis von Bakterien ist durch die Koloniehybridisierung bekannt. Dabei wird eine Membran auf Bakterienkolonien einer Petrischale gelegt, wodurch die Kolonien auf die Membran übertragen (abgestempelt) werden. Diese übertragenen Kolonien werden anschließend lysiert, die DNA denaturiert und fixiert und mit markierten Oligonukleotiden hybridisiert.

Eine raschere Identifikation der, eine Probe kontaminierenden Bakterienstämme ist mittels FISH-Gensondentechnologie mögliche, die Bakterien aufgrund ihrer rRNA-Zusammensetzung unterscheidet. Dabei werden fluoreszierende Gensonden verwenden, die in die Zellen eindringen und sich an ihre spezifische Zielstelle binden. Werden bestimmte Gensonden mit unterschiedlichen Farbstoffen gekoppelt kann aufgrund der betreffenden Farbe durch Anregung mit energiereichem Licht eine Identifikation des Bakteriums mittels mikroskopischer Auswertung erfolgen. Die DE 199 45 510 B4 beschreibt ein Verfahren zum Nachweis von Mikroorganismen in einer Probe mittels einer Nukleinsäuresonde, wobei das Verfahren folgende Schritte umfasst: a) Fixieren der in der Probe enthaltenen Mikroorganismen, b) Immobilisieren der Mikroorganismen auf einer Mikrotiterplatte c) Inkubation der auf der Mikrotiterplatte fixierten und immobilisierten Mikroorganismen mit nachweisbaren Nukleinsäuresondenmolekülen, um eine Hybridisierung herbeizuführen, d) Entfernen nicht hybridisierter Nukleinsäuresondenmoleküle und e) Detektieren und gegebenenfalls Quantifizieren der Zellen mit hybridisierten Nukleinsäuresondenmolekülen. Die Mikroorganismen werden dabei durch eine niederprozentige Paraformaldehydlösung oder eine verdünnte Formaldehydlösung in der Probe fixiert, also für Nukleinsäuresonden durchlässig gemacht.

Die WO 2006/092629 A1 beschreibt eine Detektionsvorrichtung für Bakterien, insbesondere MRSA, wobei sich die Bakteriophagen, sobald sie an ein Bakterium binden, vermehren. Die Bakteriophagen um-fassen eine Nukleinsäure, die ein fluoreszierendes Protein kodieren. Werden die multiplizierten Bakteriophagen Licht mit einer ersten Wellenlänge exponiert, steigt die Menge des Lichts der zweiten Wellenlänge. Somit kann durch eine optische Detektion der zweiten Wellenlänge nachgewiesen werden, ob Zielbakterien vorhanden sind.

Die US 2003/0039974 A1 beschreibt ein Verfahren zur Isolierung von Zielnukleinsäuren aus einer biologischen Probe, wobei das zelluläre Material mit einem chaotropen Agens lysiert wird, die Nukleinsäure denaturiert wird und an eine LNA-Probe bindet.

Die WO 2003/014382 A2 beschreibt eine Vorrichtung zur Detektion von Zielsequenzen mit Fängermolekülen.

Die WO 2009/049833 A1 beschreibt ein Verfahren zum Nachweis von Parodontitis assoziierten Bakterien mittels mikrofluidischer Vorrichtung.

Die WO2004/106547 A2 beschreibt ein Verfahren zum Nachweis von Mikroorganismen mit Hilfe eines Zielmolekül/Fängermolekül-Komplexes.

Aufgabe der vorliegenden Erfindung ist es Mikroorganismen zu kultivieren und rasch molekularbiologisch zu identifizieren.

Die Aufgabe der Erfindung wird jeweils eigenständig durch eine Hybridisierungsanordnung zum spezifischen Nachweis von Mikroorganismen in einem Kulturgefäß mit zumindest zwei Bereichen, wobei in einem ersten Bereich prokaryontische Zellen kultivierbar sind und in einem zumindest weiteren Bereich Fängermoleküle zum spezifischen Nachweis von Mikroor ganismen, welche im zumindest ersten Bereich kultivierbar sind, immobilisiert sind, sowie deren Verwendung zum spezifischen Nachweis von Mikroorganismen mit Zielmolekülen in einem Kulturgefäß mit zumindest zwei Bereichen bei Kultivierung der Mikroorganismen in zumindest einem ersten Bereich und Hybridisierung der Zielmoleküle mit Fängermolekülen in zumindest einem weiteren Bereich in ausschließlich einem Kulturgefäß sowie durch ein Verfahren zum spezifischen Nachweis von Mikroorganismen mit einer Hybridisierungsanordnung in einem Kulturgefäß mit zumindest zwei Bereichen vorzugsweise durch Hybridisierung umfassend die folgenden Schritte: a) Transferieren von Zellen bzw. Zellbestandteilen der Mikroorganismen mit Zielmolekülen von einem ersten Bereich, wo prokaryontische Zellen wachsen, in den zumindest weiteren Bereich, wo Fängermoleküle immobilisiert sind, b) Binden der Zielmoleküle an die Fängermoleküle, c) Markierung der gebundenen Zielmoleküle und d) Detektion der gebundenen Zielmoleküle. Dabei erweist sich von Vorteil, dass einerseits durch die Kultivierbarkeit der Beweis der Lebensfähigkeit der Mikroorganismen erbracht werden kann, wie dies beispielsweise in vielen Normen der Lebensmittel- bzw. Trinkwasseranalytik gefordert ist und andererseits durch den molekularbiologischen Nachweis im selben Gefäß die Kontaminationsgefahr für die Umgebung sowie die Infektionsgefahr für das Laborpersonal deutlich reduziert werden können im Vergleich zu aus dem Stand der Technik bekannten Methoden, wo das mikrobiologische Material vom Gefäß der Kultivierung in ein weiteres Gefäß zur molekularbiologischen Analyse überführt werden muss. Ferner erfolgt durch den molekularbiologischen Nachweis eine eindeutige Identifizierung der Mikroorganismen.

Vorteilhaft erweist sich auch, dass ein Kulturmedium des ersten Bereichs während der Kultivierung nicht in Kontakt mit den Fängermolekülen des zumindest weiteren Bereichs steht, weil somit eine Kontamination bzw. ein Abwaschen der Fängermoleküle vom weiteren Bereich verhindert werden kann.

In einer möglichen Ausführungsvariante kann der erste Bereich als Unterteil einer Petrischale und der weitere Bereich als Deckel einer Petrischale ausgeführt sein, wodurch ein bestehendes System für die Erfindung genutzt und entsprechend weiter entwickelt werden kann.

In einer alternativen Ausführungsvariante kann der erste Bereich der Anordnung, insbesondere Hybridisierungsanordnung, auch als Unterteil einer Platte im Mikrotiterplattenformat mit flachem Boden und der weitere Bereich als Deckel einer Platte im Mikrotiterplattenformat ausgebildet sein, wodurch ebenfalls ein standardisiertes System für die Anordnung genutzt wird und nur mehr entsprechend adaptiert werden braucht.

Im Unterteil kann ein Nährboden zur Kultivierung von Mikroorganismen angeordnet sein und im Deckel können Hybridisierungssonden als Fängermoleküle in Mustern, Arrays bzw. Spots immobilisiert sein. Dadurch kann ein für die Kultivierung von Mikroorganismen standardisiertes Gefäß verwendet werden, in welches die Hybridisierungssonden geprintet werden und somit ein dem Anwender vertrautes System zur Verfügung steht. Es können die gewohnten Standardprozeduren zum Printen der Sonden (z.B. bekannt aus US2004/043478, CA 2155634, JP2004004087, EP 1 471 355, US 6,268,128) bzw. für die Hybridisierung durchgeführt werden und man erhält als zusätzliche Anwendung die Möglichkeit der Genotypisierung, ohne neue Apparate anschaffen zu müssen.

In einer Weiterbildung der Erfindung können die zumindest zwei Bereiche durch zumindest ein Trennelement oder zumindest einen temporären Schließmechanismus voneinander getrennt sein, wodurch ein erster Bereich für die Kultivierung der Mikroorganismen zur Verfügung steht und in zumindest einem weiteren Bereich die Fängermoleküle immobilisiert werden können und somit die weitere molekularbiologische Analytik bzw. Identifikation der Mikroorganismen in diesem zumindest weiteren Bereich stattfinden kann, welcher während der Kultivierung der Mikroorganismen noch nicht mit diesem in Berührung kam.

Das zumindest eine Trennelement und/oder der zumindest eine temporäre Schließmechanismus kann eine Filtereinrichtung bzw. ein Sieb bilden bzw. aufweisen, um eventuell gelöste Nährbodenreste oder Zellen bzw. Zellbestandteile der prokaryontischen unerwünschten Zellen des ersten Bereichs abzutrennen.

Ferner kann im zumindest weiteren Bereich zumindest eine Ausnehmung angeordnet ist, in welcher die Fängermoleküle angeordnet sind, wodurch das Volumen, welches vom ersten Bereich in den zumindest weiteren Bereich auf eine kleinere Fläche reduziert wird, und somit weniger Reagenzien für den Transfer und die Identifikation der Mikroorganismen benötigt werden. Von Vorteil erweist sich zudem, dass damit auch die Nachweisgrenze der Mikroorganismen verbessert werden kann, weil der Verdünnungseffekt kleiner wird.

In einer alternativen Ausführungsvariante bzw. einer Weiterbildung kann im zumindest weiteren Bereich zumindest ein mikrofluidisches System angeordnet sein, wodurch ohne weitere Manipulation der Anordnung eine Identifikation der nachzuweisenden Mikroorganismen durchgeführt werden kann.

In einer Weiterbildung ist vorgesehen, dass zumindest eine Markierung im zumindest weiteren Bereich angeordnet ist, wodurch Positions- bzw. Orientierungskontrollsysteme geschaffen werden können.

Von Vorteil erweist sich auch die Verwendung der Anordnung, insbesondere Hybridisierungsanordnung, zum spezifischen Nachweis von Mikroorganismen mit Zielmolekülen in einem Kulturgefäß mit zumindest zwei Bereichen bei Kultivierung der Mikroorganismen in zumindest einem ersten Bereich und Bindung, insbesondere Hybridisierung, der Zielmoleküle mit Fängermolekülen in zumindest einem weiteren Bereich in ausschließlich einem Kulturgefäß, weil durch das Wegfallen der Durchführung der Analyse in einem weiteren Kulturgefäß die Kontaminationsgefahr sinkt.

Vorteilhafterweise wachsen vor dem Transfer der Zellen bzw. der Zellbestandteile im ersten Bereich Mikroorganismen und im zumindest weiteren Bereich werden Fängermoleküle immobilisiert, wodurch der Nachweis der Lebensfähigkeit der Mikroorganismen in Kombination mit einer molekularbiologischen Identifizierung erfolgen kann, die eine eindeutigere Bestimmung der Mikroorganismen im Vergleich zu morphologischen Identifizierungen zulässt.

Es wird eine Lösung für den Transfer der Zellen bzw. Zellbestandteile der Mikroorganismen mit Zielmolekülen vom ersten Bereich in den zumindest weiteren Bereich zugegeben, wodurch einerseits das Ablösen der Mikroorganismen vom ersten Bereich ermöglicht wird und andererseits auch gleichzeitig eine Lyse der Mikroorganismen erfolgen kann.

Vorteilhaft erweist sich zudem, dass durch die Lösung die Zellen gegebenenfalls unter Temperaturerhöhung lysiert werden und gegebenenfalls durch die Lösung auch die Zielmoleküle an die Fängermoleküle gebunden werden sowie die gebundenen Zielmoleküle markiert werden, sodass lediglich ein Reagens zugegeben werden muss und somit die Anzahl der Arbeitsschritte so gering wie möglich gehalten werden kann.

Zwischen den Verfahrensschritten kann der zumindest weitere Bereich gewaschen werden, wodurch eventuelle Verunreinigungen und Lösungen vorangegangener Verfahrensschritte entfernt werden, wobei sowohl nur zwischen bzw. nach ausgewählten Verfahrensschritten gewaschen werden kann als auch zwischen bzw. nach jedem Verfahrensschritt ein Waschschritt erfolgen kann.

Zum besseren Verständnis der Erfindung wird diese anhand der nachfolgenden Figuren näher erläutert.

Es zeigen jeweils in stark schematisch vereinfachter Darstellung:
- Fig. 1: einen Schnitt einer Anordnung, insbesondere Hybridisierungsanordnung;
- Fig. 2: einen Schnitt einer alternativen Ausführungsform einer Anordnung, insbesondere Hybridisierungsanordnung;
- Fig. 3: einen Schnitt einer weiteren alternativen Ausführungsform einer Anordnung, insbesondere Hybridisierungsanordnung;
- Fig. 4: eine Ausführungsform einer Anordnung, insbesondere Hybridisierungsanordnung, in Draufsicht;
- Fig. 5: eine Ausführungsform einer Anordnung, insbesondere Hybridisierungsanordnung, in Draufsicht;
- Fig. 6: eine Ausführungsform einer Hybridisierungsanordnung in Draufsicht;
- Fig. 7: einen Schnitt einer weiteren alternativen Ausführungsform einer Anordnung, insbesondere Hybridisierungsanordnung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Ferner können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Unter Mikroorganismen werden im Sinne der Erfindung Hefen, Bakterien, Viren, Algen und Pilze verstanden. Es werden Zielmoleküle dieser Organismen, die spezifisch an die Fängermoleküle binden, nachgewiesen. So können mit der erfindungsgemäßen Vorrichtung und dem erfindungsgemäßen Verfahren Mikroorganismen aus biologischen Proben, wie z.B. Blut oder Speichel, rasch nachgewiesen werden und somit mit einer entsprechenden Therapie begonnen werden. Die Vorrichtung und das Verfahren eignen sich aber auch zum Nachweis von Mikroorganismen in Lebensmittel, Wasser oder anderen Proben.

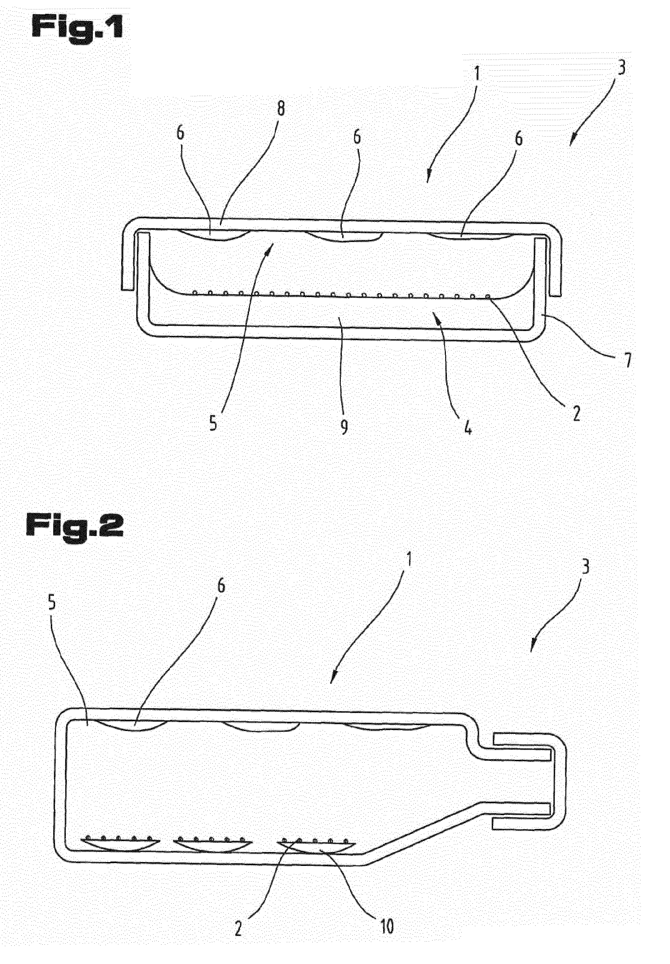

Die vorliegende Erfindung beschreibt eine Anordnung, insbesondere Hybridisierungsanordnung 1, zum spezifischen Nachweis von Mikroorganismen 2 in einem Kulturgefäß 3, wie z.B. Petrischale, Platte im Mikrotiterplattenformat, wie z.B. eine Mikrotiterplatte mit flachem Boden bzw. ohne Wells, Kulturröhrchen, Gewebekulturflasche, Rollerbottle, etc., mit zumindest zwei Bereichen 4, 5, wie sie in Fig. 1 dargestellt ist.

In zumindest einem ersten Bereich 4 eines Kulturgefäßes 3 sind prokaryontische Zellen kultivierbar. Die Zellen können sowohl in einem flüssigen Kulturmedium als auch auf einem festen Kulturboden wachsen. So kann beispielsweise eine mit Mikroorganismen 2, insbesondere Bakterien, verunreinigte Probe auf einem Nährboden 9, insbesondere Agarboden, der Anordnung, insbesondere Hybridisierungsanordnung 1, ausplattiert werden und anschließend unter entsprechenden Bedingungen inkubiert werden um ein Wachstum der nachzuweisenden Bakterien zu ermöglichen. Es können aber auch eukaryontische Zellen, welche mit Mikroorganismen 2 kontaminiert sind, kultiviert werden und die verunreinigenden Mikroorganismen 2 in der nachfolgenden Analyse nachgewiesen werden. Durch die Kultivierbarkeit ist der Beweis der Lebensfähigkeit der Mikroorganismen 2 erbracht. Dieser Nachweis der Lebensfähigkeit der Keime wird in vielen Normen gefordert, wie z.B. in der Lebensmittel- bzw. Trinkwasseranalytik.

In zumindest einem weiteren Bereich 5 desselben Kulturgefäßes 3 sind Fängermoleküle 6 zum spezifischen Nachweis von Mikroorganismen 2 angeordnet bzw. immobilisiert. Die Fängermoleküle 6 können dabei in Spots bzw. Arealen angeordnet sein, wodurch eine vermehrte Anbindung eines nachzuweisenden Zielmoleküls an einem Spot bzw. Array erfolgt und somit eine einfache, gegebenenfalls visuelle, Detektion und Identifikation der jeweiligen Mikroorganismen 2 möglich ist. Werden Petrischalen aus unbehandeltem Polystyrol als Kulturgefäße 3 verwendet, kann zur Immobilisierung der Fängermoleküle 6 im weiteren Bereich 5 beispielsweise ein Printpuffer verwendet werden.

Fängermoleküle 6 können sowohl Nukleinsäuren, insbesondere Oligonukleotide mit einer Länge von 15 bis 100 Basen von DNA, RNA, PNA, LNA, Phosphorthioat-Desoxyribonukleinsäure, Cyclohexen-Nukleinsäuren (CeNA), N3'-P5'-Phosphoramidate (NP), Tricyclo-Desoxyribonukleinsäuren (tcDNA), Morpholino-Phosphoramidate (MF) als auch Aminosäuren, insbesondere Proteine, insbesondere Antigene, Antikörper, Peptide, etc. sein.

So zeigt Fig 1. ein Kulturgefäß 3, insbesondere eine Petrischale, wo ein Array mit spezifischen Fängermolekülen 6, wie DNA-Sonden, im zumindest weiteren Bereich 5 angebracht ist, das es nach einer entsprechenden Behandlung der Kultur des ersten Bereiches 4 erlaubt in derselben Petrischale Mikroorganismen 2 nachzuweisen bzw. zu identifizieren oder zu unterscheiden.

Im speziellen zeigt Fig. 1 eine mögliche Ausführungsform der Anordnung, insbesondere Hybridisierungsanordnung 1, zum spezifischen Nachweis von Mikroorganismen 2. Die Hybridisierungsanordnung 1 umfasst ein Kulturgefäß 3, insbesondere eine Petrischale, wobei der erste Bereich 4 als Unterteil 7 der Petrischale und der weitere Bereich 5 als Deckel 8 der Petrischale ausgebildet sind. Im Unterteil 7 der Petrischale ist ein Nährboden 9 zur Kultivierung der Mikroorganismen 2 und im Deckel 8 der Petrischale sind Fängermoleküle 6, wie Oligonukleotide, insbesondere Hybridisierungssonden, angeordnet. Die Anbringung des Arrays der Fängermoleküle 6 am Deckel 8, insbesondere bei Petrischalen, ist besonders vorteilhaft, weil der Transport der Probe - also das in Kontaktbringen des Arrays mit den Mikroorganismen 2 - durch einfaches Umdrehen der Petrischale erreicht werden kann. Um einen Flüssigkeitsaustritt nach dem Wenden der Petrischale zu vermeiden kann in einer Weiterbildung eine Modifikation des Deckels 8 erfolgen, indem dieser mit einem dicht schließenden Ring, beispielsweise durch eine Dichtung, Gewinde, etc. bzw. durch einen längeren Seitenrand versehen wird.

Das Array mit den Fängermolekülen 6 kann an allen Teilen des Kulturgefäßes 3 angebracht werden, die nicht in Kontakt mit dem Kulturmedium stehen. Im Falle einer Petrischale ist das insbesondere der Deckel 8 oder die Seitenwand. Weisen die Fängermoleküle 6 im Kulturmedium eine hohe Stabilität auf, können diese auch in Kontakt mit dem Kulturmedium kommen. Allerdings kann die Anbringung des Arrays mit Fängermolekülen 6 auch außen am Kulturgefäß 3 erfolgen, wenn die Probe mit den Mikroorganismen 2 gegebenenfalls kontaminationsfrei durch eine eventuell verschließbare Öffnung in Kontakt mit dem Array gebracht wird. Wenn das Array an der Außenseite des Kulturgefäßes 3 angebracht ist, kann es als mit einer Folie oder einem Deckel abzuschließende Reaktionskammer ausgebildet sein.

Ist die Anordnung, insbesondere Hybridisierungsanordnung 1, als Platte, vorzugsweise im Mikrotiterplattenformat, ausgebildet, so ist sie wie unter Fig. 1 beschrieben ausgestaltet.

Fig. 2 zeigt eine Anordnung, insbesondere Hybridisierungsanordnung 1, in einem Kulturgefäß 3 zur Kultivierung eukaryontischer Zellen. Im ersten Bereich 4 des Kulturgefäßes 3, insbesondere der Gewebekulturflasche, wachsen eukaryontische Zellen, in oder an welchen beispielsweise Mikroorganismen 2 wachsen. Im weiteren Bereich 5 sind Fängermoleküle 6 immobilisiert, wobei auch hier die Fängermoleküle 6 sowohl Nukleinsäuren als auch Proteine sein können. Vorzugsweise sind die Fängermoleküle 6 wiederum in Arealen bzw. Spots angeordnet. Mit solchen Hybridisierungsanordnungen 1 können beispielsweise Mycoplasmeninfektionen eukaryontischer Zellkulturen nachgewiesen werden. Ein zur Kultivierung der Zellen erforderliches flüssiges Kulturmedium kommt nicht in permanente Berührung mit den Fängermolekülen 6 des weiteren Bereichs 5. Gelegentliches Spritzen des Kulturmediums in den weiteren Bereich 5 beeinträchtigt die Anordnung nicht. Das flüssige Kulturmedium soll aber nicht in kontinuierlichem Kontakt mit dem weiteren Bereich 5 mit den Fängermolekülen 6 der Anordnung stehen.

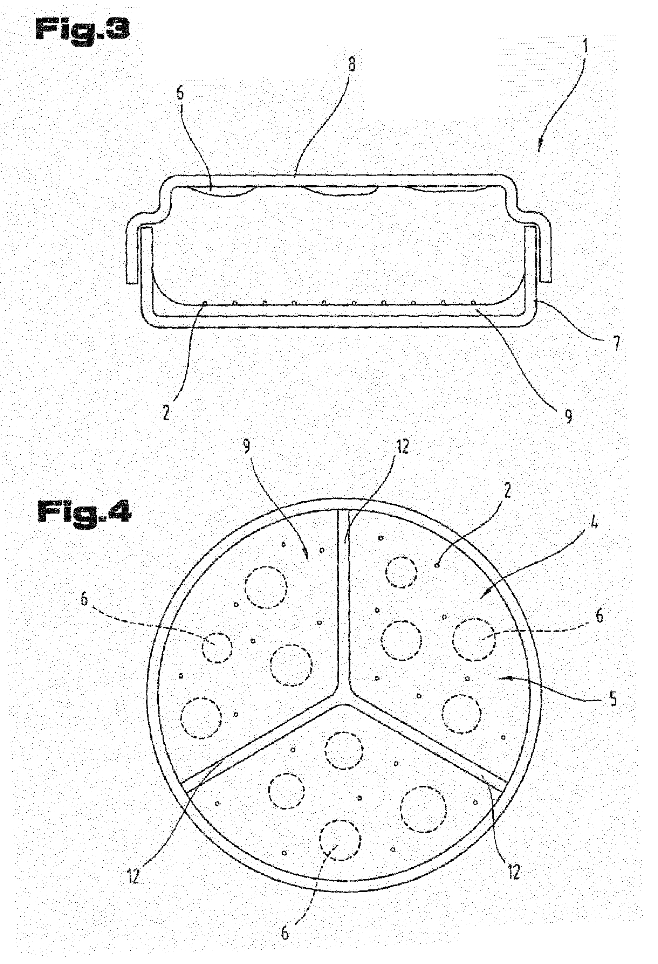

In Fig. 3 ist eine Weiterbildung der Anordnung, insbesondere Hybridisierungsanordnung 1, dargestellt. Die Petrischale aus dem Unterteil 7 mit einem Nährboden 9 weist im Deckel 8 eine Ausnehmung 11 auf. In dieser Ausnehmung 11 ist der zumindest weitere Bereich 5 angeordnet, in welchem die Fängermoleküle 6 immobilisiert sind. Durch die Verkleinerung der Fläche ist es möglich ein geringeres Volumen an Reagenzien für den Nachweis der Mikroorganismen 2 zu verwenden.

In den Fig. 4 bis 6 sind weitere Weiterbildungen der Anordnung, insbesondere Hybridisierungsanordnung 1, gezeigt, welche gemeinsam mit jener Weiterbildung aus Fig. 3 vorliegen kann.

Fig. 4 zeigt Trennelemente 12 im zumindest ersten Bereich 4, wodurch dieser erste Bereich 4 in mehrere erste Bereiche 4 unterteilt werden kann, in denen beispielsweise unterschiedliche Nährböden 9 für die Kultivierung unterschiedlicher Mikroorganismen 2 vorliegen können. Auch der zumindest weitere Bereich 5 kann durch zumindest ein Trennelement 12 in mehrere weitere gleichartige oder unterschiedliche weitere Bereiche 5 untergliedert werden, in welchen unterschiedliche Fängermoleküle 6 bzw. verschiedene Gruppen von Fängermolekülen 6 immobilisiert sein können und unterschiedliche Mikroorganismen 2 bzw. Mikroorganismengruppen identifiziert werden.

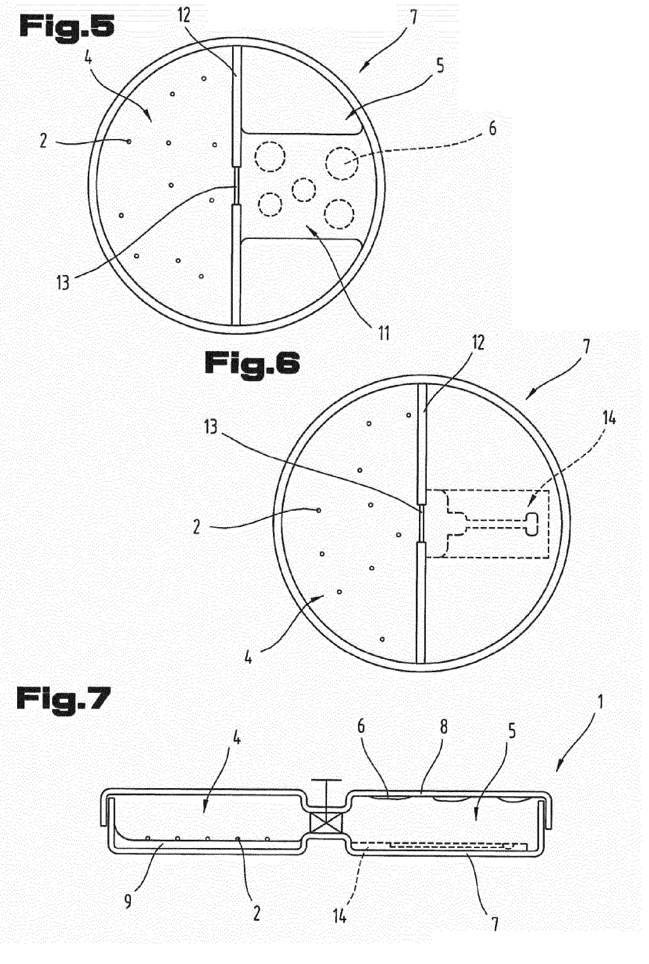

In Fig. 5 wird eine alternative Ausführungsform eines Trennelements 12 gezeigt. Dabei ist das Trennelement 12 durch einen Schließmechanismus 13 ergänzt. Das Trennelement 12 trennt dabei den ersten Bereich 4 von einem weiteren Bereich 5 eines Unterteils 7 einer Petrischale. Im Trennelement 12 ist zudem ein Schließmechanismus 13 angeordnet, der ein Übertreten von Flüssigkeit aus dem ersten Bereich 4 in den weiteren Bereich 5 bei Entfernen des Schließmechanismus 13 ermöglicht. Im weiteren Bereich 5 können die Fängermolekle 6 über die gesamte Oberfläche immobilisiert sein, oder wie in Fig. 5 dargestellt in einem kleineren Bereich des weiteren Bereichs 5 konzentriert sein, der zudem in einer Ausnehmung 11 lokalisiert sein kann. Durch die Verkleinerung der Fläche des weiteren Bereichs 5 im Vergleich zur Fläche des ersten Bereichs 4 kann das Volumen der zu verwendenden Reagenzien reduziert werden und durch Konzentration der nachzuweisenden Mikroorganismen 2 auch deren Nachweisegrenze herabgesetzt werden. In einer alternativen Ausführungsform kann die Fläche des weiteren Bereichs 5 auch durch weitere Trennelemente 12 begrenzt werden.

Sowohl das zumindest eine Trennelement 12 als auch der zumindest eine Schließmechanismus 13 können auch eine Filter- bzw. Siebfunktion ausüben, um vom Nährboden 9 gelöste Partikel oder nicht gelöste Mikroorganismenklumpen abzutrennen.

In Fig. 6 ist so wie in Fig. 5 ein erster Bereich 4 dargestellt, in welchem die Mikroorganismen 2 wachsen. Dieser erste Bereich 4 ist durch eine Trennelement 12 und in einem daran angeordneten Schließmechanismus 13 vom weiteren Bereich 5 getrennt. Im weiteren Bereich 5 ist ein mikrofluidisches System 14 angeordnet, welches zur Identifikation der Mikroorganismen 2 verwendet werden kann. Es kann beispielsweise ein mikrofluidisches System 14, wie es aus dem Stand der Technik bekannt ist, verwendet werden.

Eine weitere mögliche Ausführungsvariante ist in Fig. 7 dargestellt. Hierbei besteht die Anordnung, insbesondere Hybridisierungsanordnung 1, aus einem ersten Bereich 4 und einem weiteren Bereich 5, wobei die beiden Bereiche durch einen Schließmechanismus 13 voneinander getrennt sind. Der Schließmechanismus 13 kann als Ventil ausgebildet sein. Im ersten Bereich 4 wachsen die Mikroorganismen 2 auf einem entsprechenden Nährboden 9. Im weiteren Bereich 5 sind die Fängermoleküle 6 angeordnet. Diese können entweder am Unterteil 7 (nicht dargestellt) bzw. Deckel 8, in einer Ausnehmung 11 (nicht dargestellt) oder in einem mikrofluidischen System 14 (strichliert dargestellt) des weiteren Bereichs 5 angeordnet sein.

Im weiteren Bereich 5 können auch Kontrollsysteme angeordnet sein. So können Markierungen als Positionskontrolle und/oder zur Identifizierung der Position der Fängermoleküle 6 angeordnet sein, um die Auswertung zu erleichtern, insbesondere wenn rotationssymmetrische Hybridisierungsanordnungen 1 verwendet werden. Ferner können auch Kontrollsystems für die unterschiedlichen Verfahrensschritte angeordnet sein, die der Qualitätskontrolle dienen. Solche Systeme sind aus dem Stand der Technik, z.B. aus der WO 2003/014382 A bereits bekannt.

Mit der erfindungsgemäßen Anordnung, insbesondere Hybridisierungsanordnung 1, kann auch eine semiquantitative bzw. quantitative Auswertung erfolgen. Beispielsweise können bei kolorimetrischer Auswertung Kalibrationspunkte mit unterschiedlicher Dichte angeordnet sein, um eine semiquantitative Auswertung zu ermöglichen. Für eine quantitative Auswertung kann bei kolorimetrischer Markierung ein Scanner zum Auslesen verwendet werden und anschließend quantifiziert werden.

Der Nachweis der Mikroorganismen 2 kann aus einer Probe wie Lebensmittel, wie z.B. Milch oder Milchprodukten, Trinkwasser, Abwasser, wie z.B. Belebtschlamm, Faulschlamm oder anaeroben Schlamm, Getränke, Backwaren oder Fleischwaren, Wasser, Luft, Boden, medizinischen Probe, wie z.B. Blut, Gewebe, Urin, Fäces, Sekreten, pharmazeutischen oder kosmetischen Produkten, etc. erfolgen.

Ferner wird ein Verfahren zum spezifischen Nachweis von Mikroorganismen 1 mit einer Anordnung, insbesondere Hybridisierungsanordnung1, in einem Kulturgefäß 3 mit zumindest zwei Bereichen durch Bindung, insbesondere Hybridisierung, umfassend die folgenden Schritte: a) transferieren von Zellen bzw. Zellbestandteilen der Mikroorganismen 2 mit Zielmolekülen von einem ersten Bereich 4, wo prokaryontische Zellen wachsen, in den zumindest weiteren Bereich 5, wo Fängermoleküle 6 immobilisiert sind, b) Binden der Zielmoleküle an die Fängermoleküle 6, c) Markierung der gebundenen Zielmoleküle und d) Detektion der gebundenen Zielmoleküle, beschrieben.

Zum Transferieren der Zellen bzw. Zellbestandteile der Mikroorganismen 2 wird eine Flüssigkeit zum ersten Bereich 4 zugegeben, welche die kultivierten Mikroorganismen 2 vom festen Träger, wie z.B. Nährmedium, Kulturgefäß, Zellen, etc., löst und mit dem Array der Fängermoleküle 6 des weiteren Bereichs 5 in Kontakt bringt. Als Flüssigkeit kann ein physiologischer Puffer, wie PBS oder Tris-HCl, verwendet werden; zudem können weitere für die Lyse erforderliche Reagenzien zugesetzt sein. Es ist auch möglich nur ausgewählte Bestandteile der Lösung zuzugeben und die restlichen Bestandteile aus dem Nährboden zu lösen um auf die gewünschte Zusammensetzung der zum Transfer erforderlichen Flüssigkeit zu gelangen. Sofern Nukleinsäuren nachzuweisen sind, wird vorzugsweise eine Universallösung (ULS), die zugleich Lysepuffer ist, verwendet, um die Nukleinsäuren dem Assay sofort zugänglich zu machen. In der Universallösung ist vorzugsweise Guanidiniumthiocyanat in einer Konzentration von 1 M bis 10 M enthalten, insbesondere in einer Konzentration von 5 M. Werden allerdings Oberflächenmoleküle mit Antikörpern nachgewiesen, ist eine Lyse nicht erforderlich. Bei Verwendung der Universallösung ist es möglich die Mikroorganismenidentifikation ohne weiteren technischen Aufwand durchzuführen.

Vorzugsweise erfolgt das Ablösen der Mikroorganismen 2 vom Nährboden 9 durch eine Inkubation auf einem Schüttelinkubator. Selbstverständlich können aber auch andere Verfahren, wie z.B. Schwenken mit der Hand, Vortexen, Inkubation durch Stehen lassen, etc. verwendet werden.

Die Lyse bietet auch den Vorteil, dass die Gefahr, die vom Hantieren mit lebensfähigen hochpathogenen Keimen ausgeht, reduziert wird, da die Zellen aufgebrochen werden oder der Puffer das Wachstum zumindest stark beeinträchtigt, während bei anderen Verfahren, die eine Entnahme von lebensfähigen hochpathogenen Keimen aus den Petrischalen erfordern um sie einer weiteren Analyse zugänglich zu machen, sowohl eine Kontaminationsgefahr als auch eine Gefahrenpotential für das Personal besteht.

Gegebenenfalls kann zum Transfer der Zellen bzw. Zellbestandteilen der Mikroorganismen 2 mit Zielmolekülen von einem ersten Bereich 4, wo prokaryontische Zellen wachsen, in den zumindest weiteren Bereich 5, wo Fängermoleküle 6 immobilisiert sind, auch eine Temperaturerhöhung für eine verbesserte Lyse erforderlich sein. Es ist von Vorteil nur jenen Teil des Kulturgefäßes 3 zu erwärmen, der mit der lysierenden Lösung in Kontakt steht, aber nicht den Teil, der mit dem festen Kulturmedium, insbesondere Nährboden 9, in Kontakt ist, da sich dieser ansonsten verflüssigen könnte, was zu einer Beeinträchtigung der Analyse führen könnte. Dies kann dadurch erreicht werden, dass im Falle einer Petrischale diese zuerst umgedreht wird, sodass sie mit dem Deckel nach unten auf eine "Kochplatte" gelegt wird. Oder die Erwärmung erfolgt durch Strahlung (Mikrowelle, Infrarot, etc.), die entweder örtlich im Lysemedium fokussiert ist oder besonders stark (selektiv) im Lysemedium absorbiert wird und nicht im Kulturmedium oder Kulturgefäß 3 selbst.

Der Lysepuffer, insbesondere die Universallösung, ist bereits so konditioniert, dass dieser selbst oder nur durch Zugabe weiterer Stoffe auch eine Bindung der Zielmoleküle an die Fängermoleküle 6 ermöglicht wird. Durch das Guanidiniumthiocyanat werden stringente Bedingungen für eine Hybridisierung bei Raumtemperatur geschaffen.

In der Universallösung kann auch eine Markierung für die Zielmoleküle enthalten, wodurch bereits in einem Arbeitsschritt sowohl die Lyse der Mikroorganismen 2 als auch die Markierung der Nukleinsäure der Zielmoleküle erfolgen kann. Die Markierung kann mit allen gängigen Markern durchgeführt werden.

Es können Markierungen aus einer Gruppe umfassend Fluoreszenz-, Lumineszenz, insbesondere Chemilumineszenz-, kolorimetrische, magnetische, radioaktive, enzymatische, Haptenmarkierung bzw. durch Hybridisierung nachweisbare Nukleinsäuren verwendet werden. Insbesondere können sekundäre Fängermoleküle (Sandwich) mit direkter Markierung, wie Farbstoffmoleküle, Beads, Quantum-Dots, Partikel (magnetisch, ...), etc. verwendet werden. Alternativ können auch indirekte Verfahren eingesetzt werden, die weitere Signal-"Entwicklungsschritte" umfassen (Chemilumineszenz, Kolorimetrie (Tetramethylbenzidin (TMB), ...), etc.).

In der Universallösung ist vorzugsweise ein chaotropes Reagens, wie Guanidiniumsalze, wie Guanidiniumisothiocyanat, Formamid, Harnstoff, Perchlorat, Thiocyanat, Trichloroacetat, Nitrat, Iodid, etc. in einer Konzentration von 0,1 M bis 10 M enthalten, wodurch stringente Bedingungen für eine Hybridisierung bei Raumtemperatur geschaffen werden und andererseits die Zelllysis erfolgen kann. Durch die Verwendung von Guanidiniumthiocyanat bzw. Harnstoff wird die Anwendung teratogener Substanzen obsolet und somit Schäden bei den die Lösung manipulierenden Personen verhindert. Weiters erweist sich von Vorteil, dass in der Universallösung eine Markierung, insbesondere durch ein markiertes Oligonukleotid für die biologische Probe enthalten ist, wodurch bereits in einem Arbeitsschritt sowohl die Lyse der biologischen Probe als auch die Markierung der Nukleinsäure erfolgen kann. Die Universallösung umfasst zumindest ein chaotropes Reagens sowie zumindest ein Oligonukleotid. Die Universallösung kann beispielsweise Guanidiniumthiocyanat, Tris- HCl, DNA und ein markiertes Oligonukleotid enthalten. Ähnliche Lösungen sind beispielsweise aus der US 5,334,501 bekannt (2 bis 4 M GuSCN, vorzugsweise 3 M, 0,01 bis 0,1 M Tris (pH zwischen 6,0 bis 8,5), ein Detergens wie 0,1 bis 5 % SDS und 0,01 bis 0,1 M EDTA. Zudem können Additive, wie Träger-DNA oder RNA, Proteine, wie BSA oder Gelatine, enthalten sein. Die Stringenz kann durch Zugabe von 0 bis 10 % Formamid, gewöhnlich 5 %, verbessert werden). Weitere Beispiele für die Zusammensetzung der Universallösung sind aus der EP 0 420 260 B1 und EP 0 875 583 B1 bekannt.

Zur Herstellung von 10 ml Universallösung werden 4 ml 5M Guanidiniumthiocyanat, 1 ml 500 mM Tris HCl (pH 7,4), 0,1 ml 1 mg/ml Heringsperma DNA, 2,5 µl, 100 µM Sandwichsonde und 4,9 ml Wasser verwendet. Die Sandwich-Sonde dient zum Labeln der 16 S rRNA der biologischen Probe. Sie ist mit Biotin am 5' Ende gelabelt. Die Sandwich-Sonde weist die Sequenz 5'-CATCG AATTA AACCA CATGY TCCWC CGCTT GT-3' (SEQ ID No. 6) auf. Die Sandwichsonde bindet an die biologische Probe und weil die Sandwichsonde selbst markiert ist, wird auch die biologische Probe markiert. Vorzugsweise wird die biologische Probe mittels des Oligonukleotids SEQ ID No. 6 in der Universallösung markiert. Das Oligonukleotid SEQ ID No. 6 trägt selbst die Markierung/Label. Diese Markierung kann entweder selbst (direkt) detektiert werden, oder durch weitere Bindungsschritte (indirekt) detektiert werden.

Ein oder mehrere Waschschritte können vor/nach jedem der Verfahrensschritte a) bis c) erforderlich sein, um einerseits die Spezifität zu erhöhen oder andererseits ein Hintergrundsignal zu reduzieren.

Ein einfaches Verfahren um den Hintergrund nach dem(n) Markierungsschritt(en) zu reduzieren, ist durch Drehen des Kulturgefäßes 3 die Reaktionslösung vom Array des weiteren Bereichs 5 zu entfernen.

Bei der erfindungsgemäßen Hybridisierungsanordnung 1 wird das Koloniezählverfahren nicht behindert, weil die Spots der Fängermoleküle 6 transparent sind oder außerhalb des Gesichtsfeldes angebracht werden können.

Mit der erfindungsgemäßen Hybridisierungsanordnung 1 kann auch die Technologie des bestehenden automatisierten (optischen) Koloniezählverfahrens (automatisierte Bildanalyse) genutzt werden, z.B. durch Kolorimetrie.

### Ausführungsbeispiel

Drei Mikroliter des Printpuffers mit den entsprechenden Oligonukleotidsonden zum Nachweis von Parodontitis assoziierten Keimen, wie sie aus der WO 03/014382 bekannt sind, werden auf den Deckel 8 einer Petrischale in einem vordefinierten Muster pipettiert und anschließend bei Raumtemperatur sowie 30 Minuten bei 50°C im Trockenschrank getrocknet. Anschließend werden die geprinteten Sonden bei 120 mJ im UV-Crosslinker mit dem Deckel 8 der Petrischalen verbunden.

In das Unterteil 7 der Petrischale wird ein LB-Medium mit Agar mit folgenden Inhaltsstoffen Hefeextrakt (5 g/l), Trypton (10 g/l), Natriumchlorid (0,5-10 g/l), der pH-Wert wird auf 7 eingestellt und Agar (15 g/l), gegossen. Der Agarboden erstarrt und darauf wird eine biologische Probe mit den nachzuweisenden Mikroorganismen ausplattiert und bei 37°C über Nacht inkubiert. Durch Zugabe von 1 ml ULS auf den Agarboden und leichtes Schütteln am Schüttelinkubator werden die Mikroorganismen abgelöst. Die abgelöste Suspension wird durch Umdrehen der Petrischale vom Unterteil 7 in den Deckel 8 in der Petrischale transferiert und für 5 min auf 95°C erhitzt um die Mikroorganismen 2 zu lysieren. Anschließend werden 500 µl einer Enzymlösung zugegeben und 2 Minuten bei Raumtemperatur inkubiert. Danach wird mit 1 ml einer Waschlösung durch leichtes Schwenken gewaschen um eventuelle Agarreste vom Deckel 8 der Petrischale zu entfernen. Zuletzt werden 500 µl einer Farbreaktionslösung für 4 Minuten zugegeben und schließlich mit einigen Millilitern Wasser gewaschen. Nach dem Auftrag der jeweiligen Lösung wird der Deckel 8 geschwenkt um einerseits eine gleichmäßige Verteilung der Lösung zu erreichen und andererseits etwaige Agarreste an den Rand des Deckels 8 zu befördern. Zusammensetzungen für Enzymlösungen, Waschlösungen und entsprechende Farbreaktionslösungen sind aus dem Stand der Technik hinlänglich bekannt.

Die oben genannten Volumina werden für eine Petrischale mit einem Durchmesser von 100 mm verwendet. Die Volumina der Lösungen können auch reduziert werden, wobei allerdings eine Reduktion auf die Hälfte der Volumina erhebliche Nachteile bei der nachfolgenden Identifikation der Mikroorganismen bringt. Bei Verwendung anderer Kulturgefäße wird das Volumen entsprechend angepasst.

Die Ausführungsbeispiele zeigen mögliche Ausführungsvarianten der Anordnung, insbesondere Hybridisierungsanordnung 1, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

In der Fig. 2 ist eine weitere und gegebenenfalls für sich eigenständige Ausführungsform der Anordnung, insbesondere Hybridisierungsanordnung 1, gezeigt, wobei wiederum für gleiche Teile gleiche Bezugszeichen bzw. Bauteilbezeichnungen wie in der vorangegangenen Fig. 1 verwendet werden. Um unnötige Wiederholungen zu vermeiden, wird auf die detaillierte Beschreibung in den vorangegangenen Fig. 1 hingewiesen bzw. Bezug genommen.

Der Ordnung halber sei abschließend darauf hingewiesen, dass zum besseren Verständnis des Aufbaus der Anordnung, insbesondere Hybridisierungsanordnung 1, diese bzw. deren Bestandteile teilweise nicht maßstäblich und/oder vergrößert und/oder verkleinert dargestellt wurden.

Die den eigenständigen erfinderischen Lösungen zugrundeliegende Aufgabe kann der Beschreibung entnommen werden.

Vor allem können die einzelnen in den Fig. 3; 4 bis 6; und 7 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

### Bezugszeichenaufstellung

- 1: Hybridisierungsanordnung
- 2: Mikroorganismen
- 3: Kulturgefäß
- 4: Erster Bereich
- 5: Weiterer Bereich

- 6: Fängermolekül
- 7: Unterteil
- 8: Deckel
- 9: Nährboden
- 10: Zellen

- 11: Ausnehmung
- 12: Trennelement
- 13: Schließmechanismus
- 14: Mikrofluidisches System

## Patentansprüche

1. Hybridisierungsanordnung (1)für den spezifischen Nachweis von Mikroorganismen (2) in einem Kulturgefäß (3) mit zumindest zwei Bereichen, **dadurch gekennzeichnet, dass** in zumindest einem ersten Bereich (4), der als Unterteil (7) einer Petrischale mit einem Nährboden (9) zur Kultivierung von Mikroorganismen (2) ausgebildet ist, prokaryontische Zellen kultivierbar sind und in einem zumindest weiteren Bereich (5), der als Deckel (8) einer Petrischale ausgeführt ist, Fängermoleküle (6) zum spezifischen Nachweis von Mikroorganismen (2), welche im zumindest ersten Bereich (4) kultivierbar sind, immobilisiert sind.

2. Anordnung, insbesondere Hybridisierungsanordnung (1), nach Anspruch 1, **dadurch gekennzeichnet, dass** ein Kulturmedium des ersten Bereichs (4) während der Kultivierung nicht in Kontakt mit den Fängermolekülen (6) des zumindest weiteren Bereichs (5) steht.

3. Hybridisierungsanordnung (1) nach einem der Ansprüche 1 oder 2, **dadurch gekennzeichnet, dass** als Fängermoleküle (6) Hybridisierungssonden in Form von Mustern, Arrays, Spots im Deckel (8) immobilisiert sind.

4. Hybridisierungsanordnung (1) nach einem der Ansprüche 1 bis 3, **dadurch gekennzeichnet, dass** jeweils im zumindest ersten (4) bzw. weiteren Bereich (5) und/oder die zumindest zwei Bereiche durch zumindest ein Trennelement (12) und/oder zumindest einen temporären Schließmechanismus (13) voneinander getrennt sind.

5. Hybridisierungsanordnung (1) nach Anspruch 4, **dadurch gekennzeichnet, dass** das zumindest eine Trennelement (12) und/oder der zumindest eine temporäre Schließmechanismus (13) eine Filtereinrichtung bzw. ein Sieb bilden bzw. angeordnet ist.

6. Hybridisierungsanordnung (1) nach einem der Ansprüche 1 bis 5, **dadurch gekennzeichnet, dass** im zumindest weiteren Bereich (5) zumindest eine Ausnehmung (11) angeordnet ist, in welcher die Fängermoleküle (6) angeordnet sind.

7. Hybridisierungsanordnung (1) nach einem der Ansprüche 1 bis 6, **dadurch gekennzeichnet, dass** im zumindest weiteren Bereich (5) zumindest ein mikrofluidisches System (14) angeordnet ist.

8. Hybridisierungsanordnung (1) nach einem der Ansprüche 1 bis 7, **dadurch gekennzeichnet, dass** zumindest eine Markierung im zumindest weiteren Bereich (5) angeordnet ist.

9. Verwendung einer Hybridisierungsanordnung (1) nach einem der Ansprüche 1 bis 8 zum spezifischen Nachweis von Mikroorganismen (2) mit Zielmolekülen in einem Kulturgefäß (3) mit zumindest zwei Bereichen bei Kultivierung der Mikroorganismen (2) in zumindest einem ersten Bereich (4) und Bindung, insbesondere Hybridisierung, der Zielmoleküle mit Fängermolekülen (6) in zumindest einem weiteren Bereich (5) in ausschließlich einem Kulturgefäß (3).

10. Verfahren zum spezifischen Nachweis von Mikroorganismen (2) mit einer Hybridisierungsanordnung (1) in einem Kulturgefäß (3) mit zumindest zwei Bereichen wobei in zumindest einem ersten Bereich (4), der als Unterteil (7) einer Petrischale mit einem Nährboden (9) zur Kultivierung von Mikroorganismen (2) ausgebildet ist, prokaryontische Zellen kultivierbar sind und in einem zumindest weiteren Bereich (5), der als Deckel (8) einer Petrischale ausgeführt ist, Fängermoleküle (6) zum spezifischen Nachweis von Mikroorganismen (2), welche im zumindest ersten Bereich (4) kultivierbar sind, immobilisiert sind umfassend die folgenden Schritte: a) Transferieren von Zellen bzw. Zellbestandteilen der Mikroorganismen (2) mit Zielmolekülen vom ersten Bereich (4), wo prokaryontische Zellen wachsen, in den zumindest weiteren Bereich (5), wo Fängermoleküle (6) immobilisiert sind, b) Binden der Zielmoleküle an die Fängermoleküle (6), c) Markierung der gebundenen Zielmoleküle und d) Detektion der gebundenen Zielmoleküle.

11. Verfahren nach Anspruch 10, **dadurch gekennzeichnet, dass** vor dem Transfer der Zellen bzw. der Zellbestandteile die Mikroorganismen (2) im ersten Bereich (4) wachsen und im zumindest weiteren Bereich (5) Fängermoleküle (6) immobilisiert werden.

12. Verfahren nach Anspruch 10 oder 11, **dadurch gekennzeichnet, dass** eine Lösung für den Transfer der Zellen bzw. Zellbestandteile der Mikroorganismen (2) vom ersten Bereich (4) in den zumindest weiteren Bereich (5) zugegeben wird.

13. Verfahren nach einem der Ansprüche 10 bis 12, **dadurch gekennzeichnet, dass** durch die Lösung die Zellen, gegebenenfalls unter Temperaturerhöhung, lysiert werden und gegebenenfalls durch die Lösung auch die Zielmoleküle an die Fängermoleküle (6) gebunden werden sowie die gebundenen Zielmoleküle markiert werden.

14. Verfahren nach einem der Ansprüche 10 bis 13, **dadurch gekennzeichnet, dass** zwischen zumindest einem der Schritte a) bis d) der zumindest weitere Bereich (5) mit einer Waschlösung und/oder Wasser gewaschen wird.

## Claims

1. A hybridisation arrangement (1) for the specific detection of microorganisms (2) in a culture vessel (3) having at least two regions, **characterised in that** prokaryotic cells can be cultivated in at least one first region (4), which is formed as the lower part (7) of a Petri dish with a culture medium (9) for the cultivation of microorganisms (2), and capture molecules (6) for the specific detection of microorganisms (2), which can be cultivated in the at least first region (4), are immobilized in an at least further region (5), which is embodied as a lid (8) of a Petri dish.

2. The arrangement, in particular hybridisation arrangement (1), according to claim 1, **characterised in that** a culture medium of the first region (4) is not in contact with the capture molecules (6) of the at least further region (5) during the cultivation.

3. The hybridisation arrangement (1) according to one of claims 1 or 2, **characterised in that** hybridisation probes in the form of patterns, arrays, or spots are immobilised in the lid (8) as capture molecules (6).

4. The hybridisation arrangement (1) according to one of claims 1 to 3, **characterised in that** in each of the at least first (4) and further region (5) and/or the at least two regions are separated from one another by at least one separation element (12) and/or at least one temporary closing mechanism (13).

5. The hybridisation arrangement (1) according to claim 4, **characterised in that** the at least one separation element (12) and/or the at least one temporary closing mechanism (13) form a filter means or a screen or is arranged.

6. The hybridisation arrangement (1) according to one of claims 1 to 5, **characterised in that** at least one recess (11) is arranged in the at least further region (5), in which recess the capture molecules (6) are arranged.

7. The hybridisation arrangement (1) according to one of claims 1 to 6, **characterised in that** at least one microfluidic system (14) is arranged in the at least further region (5).

8. The hybridisation arrangement (1) according to one of claims 1 to 7, **characterised in that** at least one marker is arranged in the at least further region (5).

9. Use of a hybridisation arrangement (1) according to one of claims 1 to 8 for the specific detection of microorganisms (2) comprising target molecules in a culture vessel (3) having at least two regions, the microorganisms (2) being cultivated in at least one first region (4) and the target molecules being bound, in particular hybridised, with capture molecules (6) in at least one further region (5) in exclusively one culture vessel (3).

10. A method for the specific detection of microorganisms (2) using a hybridisation arrangement (1) in a culture vessel (3) having at least two regions, wherein prokaryotic cells can be cultivated in at least one first region (4), which is formed as the lower part (7) of a Petri dish with a culture medium (9) for the cultivation of microorganisms (2), and capture molecules (6) for the specific detection of microorganisms (2), which can be cultivated in the at least first region (4), are immobilized in an at least further region (5), which is embodied as a lid (8) of a Petri dish, said method comprising the following steps: a) transferring cells or cell parts of the microorganisms (2) comprising target molecules from the first region (4), where prokaryotic cells are growing, into the at least further region (5), where capture molecules (6) are immobilised, b) binding the target molecules to the capture molecules (6), c) marking the bound target molecules, and d) detecting the bound target molecules.

11. The method according to claim 10, **characterised in that**, before the transfer of the cells or of the cell parts, the microorganisms (2) grow in the first region (4) and capture molecules (6) are immobilised in the at least further region (5).

12. The method according to claim 10 or 11, **characterised in that** a solution is added for the transfer of the cells or cell parts of the microorganisms (2) from the first region (4) into the at least further region (5).

13. The method according to one of claims 10 to 12, **characterised in that**, as a result of the solution, the cells are lysed, possibly with a temperature increase, and the target molecules are possibly also bound to the capture molecules (6) by the solution, and the bound target molecules are marked.

14. The method according to one of claims 10 to 13, **characterised in that** the at least further region (5) is washed with a washing solution and/or water between at least one of steps a) to d).

## Revendications

1. Dispositif d'hybridation (1) pour la détection spécifique de micro-organismes (2) dans un récipient de culture (3) avec au moins deux zones, **caractérisé en ce que**, dans au moins une première zone (4), qui est conçue comme une partie inférieure (7) d'une boîte de Pétri avec un fond nutritif (9) pour la culture de micro-organismes (2), des cellules procaryotes peuvent être cultivées et, dans au moins une zone supplémentaire (5), qui est conçue comme un couvercle (8) d'une boîte de Pétri, des molécules de capture (6) sont immobilisées pour la détection spécifique de micro-organismes (2) qui peuvent être cultivés dans au moins la première zone (4).

2. Dispositif, plus particulièrement dispositif d'hybridation (1) selon la revendication 1, **caractérisé en ce qu'**un milieu de culture de la première zone (4) n'entre pas en contact durant la culture avec les molécules de capture (6) de l'au moins une zone supplémentaire (5).

3. Dispositif d'hybridation (1) selon l'une des revendications 1 ou 2, **caractérisé en ce que**, en tant que molécules de capture (6), des sondes d'hybridation sont immobilisées sous la forme de motifs, de matrices, de points dans le couvercle (8).

4. Dispositif d'hybridation (1) selon l'une des revendications 1 à 3, **caractérisé en ce que**, dans au moins une première zone (4) ou une zone supplémentaire (5) et/ou les au moins deux zones sont séparées entre elles par au moins un élément de séparation (12) et/ou au moins un mécanisme de fermeture temporaire (13).

5. Dispositif d'hybridation (1) selon la revendication 4, **caractérisé en ce que** l'au moins un élément de séparation (12) et/ou l'au moins un mécanisme de fermeture temporaire (13) constituent ou comprennent un dispositif de filtration ou un tamis.

6. Dispositif d'hybridation (1) selon l'une des revendications 1 à 5, **caractérisé en ce que**, dans au moins une zone supplémentaire (5), se trouve au moins un évidement (11) dans lequel sont disposées les molécules de capture (6).

7. Dispositif d'hybridation (1) selon l'une des revendications 1 à 6, **caractérisé en ce que**, dans l'au moins une zone supplémentaire (5), se trouve au moins un système micro-fluidique (14).

8. Dispositif d'hybridation (1) selon l'une des revendications 1 à 7, **caractérisé en ce qu'**au moins un marquage se trouve dans l'au moins une zone supplémentaire (5).

9. Utilisation d'un dispositif d'hybridation (1) selon l'une des revendications 1 à 8 pour la détection spécifique de micro-organismes (2) avec des molécules cibles dans un récipient de culture (3) avec au moins deux zones de culture des micro-organismes (2) dans au moins une première zone (4) et une liaison, plus particulièrement une hybridation des molécules cibles avec des molécules de capture (6) dans au moins une zone supplémentaire (5) dans exclusivement un récipient de culture (3).

10. Procédé de détection spécifique de micro-organismes (2) avec un dispositif d'hybridation (1) dans un récipient de culture (3) avec au moins deux zones, des cellules procaryotes pouvant être cultivées dans au moins une première zone (4), qui est conçue comme une partie inférieure (7) d'une boîte de Pétri avec un fond nutritif (9) pour la culture de micro-organismes (2) et, dans au moins une zone supplémentaire (5), qui est conçue comme un couvercle (8) d'une boîte de Pétri, des molécules de capture (6) étant immobilisées pour la détection de micro-organismes (2), qui peuvent être cultivés dans au moins une première zone (4), comprenant les étapes suivantes : a) transfert des cellules ou des composants des cellules des micro-organismes (2) avec des molécules cibles, de la première zone (4), où croissent des cellules procaryotes, vers l'au moins une zone supplémentaire (5), où des molécules de capture (6) sont immobilisées, b) liaison des molécules cibles aux molécules de capture (6), c) marquage des molécules cibles et d) détection des molécules cibles liées.

11. Procédé selon la revendication 10, **caractérisé en ce que**, avant le transfert des cellules ou des composants des cellules, les micro-organismes (2) croissent dans la première zone (4) et des molécules de capture (6) sont immobilisées dans l'au moins une zone supplémentaire (5).

12. Procédé selon la revendication 10 ou 11, **caractérisé en ce qu'**une solution est ajoutée, pour le transfert des cellules ou des composants des cellules des micro-organismes (2) de la première zone (4) vers l'au moins une zone supplémentaire (5).

13. Procédé selon l'une des revendications 10 à 12, **caractérisé en ce que** la solution permet de lyser les cellules, le cas échéant avec une augmentation de la température, et, le cas échéant, la solution permet également de lier les molécules cibles aux molécules de capture (6) et de marquer les molécules cibles liées.

14. Procédé selon l'une des revendications 10 à 13, **caractérisé en ce que**, entre au moins une des étapes a) à d), l'au moins une zone supplémentaire (5) est lavée avec une solution de lavage et/ou avec de l'eau.
